# EUROPÄISCHE PATENTSCHRIFT

(11) **EP 0 799 894 B1**
(45) Veröffentlichungstag und Bekanntmachung des Hinweises auf die Patenterteilung: **19.05.2004**
(21) Anmeldenummer: 97101401.4
(22) Anmeldetag: 30.01.1997
(51) Int. Cl.: C12P 13/00, C12P 17/00, C12N 11/12, C12N 9/88

(54) **Verfahren zur Herstellung von (S)-Cyanhydrinen**
Process for the preparation of (S)-Cyanhydrines
Procédé de préparation de (S)-cyanhydrines

(30) Priorität: 09.02.1996 DE 19604715
(43) Veröffentlichungstag der Anmeldung: 08.10.1997
(73) Patentinhaber: Degussa AG, 40474 Düsseldorf (DE)
(72) Erfinder: Effenberger, Franz, Prof. Dr., 70597 Stuttgart (DE); Wajant, Harald, Dr., 70771 Leinfelden-Echterdingen (DE); Förster, Siegfried, Dr., 70563 Stuttgart (DE); Roos, Jürgen, 70193 Stuttgart (DE)

(56) Entgegenhaltungen:
- EP-A- 0 276 375
- EP-A- 0 326 063
- EP-A- 0 350 908
- EP-A- 0 446 826
- EP-A- 0 539 767
- EP-A- 0 547 655
- EP-A- 0 632 130
- WO-A-97/03204
- DE-C- 4 041 896
- FR-A- 2 222 383
- US-A- 3 947 325
- US-A- 5 177 242
- DATABASE WPI Section Ch, Week 197803 Derwent Publications Ltd., London, GB; Class A96, AN 1978-05694A XP002118115 & JP 52 145592 A (KANSAI PAINT CO LTD), 3. Dezember 1977 (1977-12-03)
- HUGHES J ET AL: "PURIFICATION, CHARACTERIZATION, AND CLONING OF ALPHA -HYDROXYNITRILE LYASE FROM CASSAVA (MANIHOT ESCULENTA CRANTZ)" ARCHIVES OF BIOCHEMISTRY AND BIOPHYSICS, Bd. 311, Nr. 2, 1. Juni 1994 (1994-06-01), Seiten 496-502, XP002022207 ISSN: 0003-9861
- EFFENBERGER F ET AL: "ENZYME-CATALYZED SYNTHESIS OF (S)-CYANOHYDRINS AND SUBSEQUENT HYDROLYSIS TO (S)-ALPHA-HYDROXY-CARBOXYLIC ACIDS" TETRAHEDRON LETTERS, Bd. 31, Nr. 9, 1. Januar 1990 (1990-01-01), Seiten 1249-1252, XP002039224 ISSN: 0040-4039
- WAJANT, HARALD (1) ET AL: "Enantioselective synthesis of aliphatic (S)-cyanohydrins in organic solvents using hydroxynitrile lyase from Manihot esculenta." ANNALS OF THE NEW YORK ACADEMY OF SCIENCES, (1996 OCT 12) 799 771-6. JOURNAL CODE: 5NM. , XP002118114
- WAJANT H. ET AL: 'Aceton cyanhydrin lysase from Manihot esculenta (cassava) is serologically distinct from other hydroxynitrile lyases' PLANT SCIENCE Bd. 108, Nr. 1, 1995, Seiten 1 - 11
- DATABASE BREDA [Online] EC 4.1.2.37 'Acetone-cyanhydrin lyase'

## Beschreibung

Die Erfindung bezieht sich auf ein Verfahren zur Herstellung von (S)-Cyanhydrinen der allgemeinen Formel I 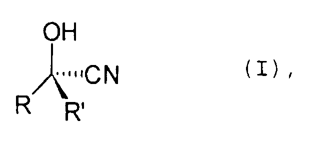 worin die Reste R und R' jeweils unabhängig voneinander bedeuten:
- Wasserstoff;
- einen substituierten oder unsubstituierten, linearen oder verzweigten, gesättigten Alkylrest mit 1 bis 18 C-Atomen, der als Substituenten einen oder mehrere Amin-, Imin-, Hydroxy-, C₁-C₈-Alkoxy, Halogen-, Carboxyl-, C₃-C₂₀-Cycloalkylrest und/oder einen ggf. N,O,S-heteroatomsubstituierten aromatischen Ring mit bis zu 22 C-Atomen aufweisen kann, wobei die zyklischen Substituenten selbst ein- oder mehrfach mit Halogen, Hydroxy und/oder linearen oder verzweigten C₁-C₈-Alkyl oder C₂-C₈-Alkenyl oder C₂-C₈-Alkinyl substituiert sein können;
- einen substituierten oder unsubstituierten, linearen oder verzweigten, ein- oder mehrfach ungesättigten Alkenyl- oder Alkinylrest mit 2 bis 18 C-Atomen, der als Substituenten einen oder mehrere Amin-, Imin-, Hydroxy-, C₁-C₈-Alkoxy, Halogen-, Carboxyl-, C₃-C₂₀-Cycloalkylrest und/oder einen ggf. N,O,S-heteroatomsubstituierten aromatischen Ring mit bis zu 22 C-Atomen aufweisen kann, wobei die zyklischen Substituenten selbst ein- oder mehrfach mit Halogen, Hydroxy und/oder linearen oder verzweigten C₁-C₈-Alkyl oder C₂-C₈-Alkenyl oder C₂-C₈-Alkinyl substituiert sein können;
- einen substituierten oder unsubstituierten aromatischen oder heteroaromatischen Rest mit 5 bis 22 Ringatomen, wobei bis zu 4 der Ringkohlenstoffatome durch N, O und/oder S ersetzt sein können, und wobei der Rest als Substituent einen oder mehrere Amin-, Imin-, Hydroxy-, C₁-C₈-Alkoxy-, Aryloxy-, Halogen-, Carboxy- und/oder linearen oder verzweigten, gesättigten oder einfach oder mehrfach ungesättigten Alkylrest mit ein bis 22 C-Atomen aufweisen kann, und wobei wenigstens zwei der Substituenten am Ring zu einem Zyklus zusammengeschlossen sein können;
mit der Maßgabe, daß R' und R nicht gleichzeitig Wasserstoff bedeuten;
durch enzymkatalysierte Umsetzung von Carbonylverbindungen der allgemeinen Formel II 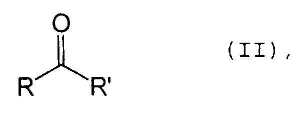 worin
R und R' die bei Formel (I) angegebene Bedeutung besitzen,
mit Blausäure oder einer Blausäure oder CN⁻ für die Umsetzung liefernden Substanz in Gegenwart einer die Umsetzung katalysierenden Menge einer immobilisierten (S)-Oxynitrilase.

Ausgehend von chiralen Cyanhydrinen ist eine Vielzahl wichtiger Substanzklassen optisch aktiver Verbindungen, wie z. B. α-Aminoalkohole, α-Hydroxyaldehyde und α-Hydroxycarbonsäuren, gut zugänglich. Methoden zur Herstellung optisch aktiver (S)-Cyanhydrine sind in der Literatur beschrieben.

Innerhalb der chemischen Methoden zur Synthese optisch aktiver Cyanhydrine spielt die enantioselektive Addition von Trimethylsilylcyanid in Gegenwart chiraler Katalysatoren eine bedeutende Rolle.

Gemäß H. Minamikawa, S. Hayakawa, T. Yamada, N. Iwasawa, K. Narasaka, Bull. Chem. Soc. Jpn. 61 (1988), 4379 und K. Narasaka, T. Yamada, H. Minamikawa, Chem. Lett. 1987, 2073 entstehen die (R)-Cyanhydrine ausgehend von einigen aliphatischen und aromatischen Aldehyden in guten chemischen und optischen Ausbeuten (61 - 93 % ee). Die Autoren geben an, daß bei Verwendung des entsprechend anderen Enantiomeren des Katalysators die (S)-Cyanhydrine gebildet werden.

Als weiterer chiraler Katalysator wurde gemäß M. Hayashi, T. Matsuda, N. Oguni, J. Chem. Soc. Chem. Commun. 1990, 1364 Titantetraisopropanolat mit L(+)-Diisopropyltartrat oder mit chiralen Schiffschen Basen eingesetzt (M. Hayashi, Y. Miyamoto, T. Inoue, N. Oguni, J. Org. Chem. 58 (1993), 1515). Je nach verwendetem Katalysator entstanden sowohl aromatische als auch aliphatische (S)- bzw. (R)-Cyanhydrine in meist nur unbefriedigenden Enantiomerenüberschüssen von 22 - 96 % ee.

Mit einem Bisoxazolin-Magnesium-Komplex als chiralem Katalysator gelang es gemäß E. J. Corey, Z. Wang, Tetrahedron Lett. 34 (1993), 4001, einige aliphatische (S)-Cyanhydrine durch Cyanosilylierung in sehr guten chemischen und optischen Ausbeuten (ee-Werte bis 95 % für Heptanal) zu bekommen.

Gute Enantiomerenüberschüsse lassen sich auch bei der diastereoselektiven Cyanosilylierung von mit 2(R),4(R)-Pentandiol acetalisierten Aldehyden gewinnen. Gemäß J. D. Elliot, V. Choi, W. P. Johnson, J. Org. Chem. 48 (1983), 2294 wurde hierbei für das (R)-Mandelonitril ein ee-Wert von 93 % bei 97 % Ausbeute erreicht. Verwendet man das entsprechende 2(S),4(S)-Pentandiol, so entsteht auf gleiche Weise das (S)-Cyanhydrin.

Auch optisch aktive geschützte α-Aminoaldehyde (M. T. Reetz, M. W. Drewes, K. Harms, W. Reif, Tetrahedron Lett. 29 (1988), 3295; J. Herranz, J. Castro-Pichel, T. Gracia-Lopez, Synthesis 1989, 703) bzw. α-Hydroxyaldehyde (M. T. Reetz, K. Kesseler, A. Jung, Angew. Chem. 97 (1985), 989) lassen sich unter Lewis-Säure-Katalyse mit Trimethylsilylcyanid oder Tributylzinncyanid unter mäßiger bis guter Diastereoselektivität in die entsprechenden β-Amino-α-hydroxy- bzw. α,β-Dihydroxynitrile überführen.

Aus F. Effenberger, U. Stelzer, Angew. Chem. 103 (1991), 866 und F. Effenberger, U. Stelzer, Chem. Ber. 126 (1993), 779 ist eine weitere Möglichkeit zur Synthese von (S)-Cyanhydrinen ausgehend von den (R)-Cyanhydrinen bekannt.

Die (R)-Cyanhydrine werden dabei sulfonyliert und danach mit Kaliumacetat unter S_{N}2-Bedingungen umgesetzt. Die Acetylgruppe entfernt man im wäßrig Sauren. Bei aliphatischen Cyanhydrinen laufen alle Stufen völlig racemisierungsfrei ab, lediglich bei aromatischen Cyanhydrinen tritt teilweise Racemisierung ein. Diese Methode kann vor allem für solche Verbindungen eingesetzt werden, die wegen des begrenzten Substratspektrums der (S)-Oxynitrilasen seither nicht direkt zugänglich waren.

Ebenfalls unter Inversion von (R)-Cyanhydrinen läuft die Mitsunobu-Reaktion ab (E. Warmerdam, J. Brussee, C. G. Kruse, A. van der Gen, Tetrahedron 49 (1993), 1063).

Insgesamt gesehen ist es jedoch in jedem Falle erstrebenswert, die chemischen Methoden durch enzymkatalysierte Methoden zu ersetzen.

(R)-Cyanhydrine sehr unterschiedlicher Struktur sind über die mit Hydroxynitril-Lyase aus Bittermandeln (PaHNL) [EC 4.1.2.10] katalysierte Addition von Blausäure an Aldehyde in sehr guten optischen Ausbeuten zugänglich (F. Effenberger, Angew. Chem. 1994, 106, 1609 - 1619). Da das Enzym auch in technischen Mengen leicht zugänglich ist, bietet dieses Verfahren den einfachsten Zugang zu (R)-Cyanhydrinen.

Wesentlich verschieden davon sind jedoch die Verhältnisse für die Enzym-katalysierte Darstellung von (S)-Cyanhydrinen. Als bislang am erfolgreichsten hat sich hierbei die Verwendung der Hydroxynitril-Lyase aus Sorghum bicolor (SbHNL) [EC 4.1.2.11] als Katalysator erwiesen (F. Effenberger, B. Hörsch, S. Förster, T. Ziegler, Tetrahedron Lett. 1990, 31, 1249 - 1252; U. Niedermeyer, M.-R. Kula, Angew. Chem. 1990, 102, 423 - 425; Angew. Chem. Int. Ed. Engl. 1990, 29, 386 - 387; M.-R. Kula, U. Niedermeyer, I. M. Stuertz, EP-B 350 908, 1990; DE-B 38 23 866 (Chem. Abstr. 1990, 113, 57462h)), ein Enzym, das aus Hirsekeimlingen inzwischen auch in Mengen gewonnen werden kann, die für synthetische Anwendungen ausreichen. Neben der schwierigeren Zugänglichkeit ist das im Vergleich zur PaHNL deutlich eingeschränkte Substratspektrum der SbHNL, die nur aromatische und heteroaromatische Aldehyde als Substrate akzeptiert, ein gravierender Nachteil für die Anwendung dieses Enzyms.

H. Wajant et al. (Plant Science 108, 1995, 1-11 beschreibt ein Verfahren zur Herstellung von (S)- Cyanhydrinen ausgehend von Carbonylverbindungen und Blausäure unter Verwendung von auf avicel-Cellulose immobilisierter S-Acetone cyanohydrin lyase (=S-Hydroxynitril Lyase bzw. S-Oxynitrilase) aus Manihot esculenta (EC 4.1.2.37).

Angesichts des hierin genannten und diskutierten Standes der Technik war es mithin eine Aufgabe der Erfindung, ein Verfahren der eingangs erwähnten Art so fortzubilden, daß die Gewinnung von (S)-Cyanhydrinen in hoher Ausbeute und vor allem in hoher Enantiomerenreinheit gestattet. Ferner war es Aufgabe der Erfindung, ein enzymatisches Verfahren bereitzustellen, das es gestattet, ein möglichst breites Substratspektrum an Ausgangsverbindungen zur gewünschten Zielverbindung umzusetzen.

Noch eine Aufgabe der Erfindung ist darin zu sehen, Enzyme einzusetzen, die möglichst einfach in ausreichenden Mengen und in hoher Aktivität zur Verfügung stehen.

Gelöst werden diese sowie weitere nicht im einzelnen angegebene Aufgaben bei einem Verfahren der eingangs genannten Art durch das kennzeichnende Merkmal des Anspruches 1. Zweckmäßige Verfahrensvarianten werden in den auf Anspruch 1 rückbezogenen Ansprüchen unter Schutz gestellt.

Dadurch, daß als Träger für die immobilisierte (S)-Oxynitrilase Nitrocellulose eingesetzt wird, gelingt eine deutliche Steigerung sowohl der Ausbeute an (S)-Cyanhydrinen als auch des Enantiomerenüberschusses in nicht ohne weiteres vorhersehbarer Weise, verglichen mit einem herkömmlichen Cellulose-Trägermaterial.

Bei der im Rahmen der Erfindung als Träger für die Immobilisierung der (S)-Oxynitrilase angewandten Nitrocellulose handelt es sich um kommerziell erhältliche Ware. Dabei ist an dieser Stelle ausdrücklich darauf hinzuweisen, daß die von der Fachwelt üblicherweise gebrauchte Bezeichnung "Nitrocellulose" falsch ist, da es sich bei Nitrocellulose um Salpetersäureester der Cellulose handelt, welche keine Nitrogruppen enthalten.

Die Salpetersäureester der Cellulose, welche für die Erfindung geeignet sind, können unterschiedliche Veresterungsgrade aufweisen, die über den Stickstoffgehalt der Cellulose bestimmt werden können. Zu den erfindungsgemäß einsetzbaren Celluloseestern gehören insbesondere das Mononitrat, das Dinitrat oder das Trinitrat sowie Zwischenstufen und Mischungen der vorgenannten Substanzen. Besonders geeignete Nitrocellulosen sind diejenigen, welche beispielsweise in der Biochemie als Blotting-Materialien beschrieben sind (H. Holtzhauer in Biochemische Labormethoden, Arbeitsvorschriften und Tabellen, Heidelberger Taschenbücher, Springer Verlag, Berlin).

Die durch an Nitrocellulose immobilisierte (S)-Oxynitrilase katalysierte Umsetzung von Carbonylverbindungen der allgemeinen Formel II zu (S)-Cyanhydrinen der allgemeinen Formel I wird zweckmäßig in einem organischen Lösungsmittel durchgeführt. Die Wahl des organischen Lösungsmittels ist im wesentlichen unkritisch, besonders bevorzugt sind solche Lösungsmittel, welche die Edukte lösen und eine leichte Isolierung der Produkte zulassen. Entsprechende organische Lösungsmittel sind dem Fachmann geläufig, besonders zweckmäßig wird als organisches Lösungsmittel Diisopropylether eingesetzt, es kommen jedoch auch alle weiteren üblicherweise eingesetzten Ether, wie Diethylether, Tetrahydrofuran, etc., oder auch Lösungsmittel, wie Essigester, zum Einsatz.

Grundsätzlich ist es von Vorteil, wenn die Lösungsmittel hoch rein eingesetzt werden. Eine absolute Wasserfreiheit ist nicht notwendig, im Gegenteil, es ist von besonderem Vorteil, wenn das organische Lösungsmittel Spuren von Wasser enthält. Dabei hat sich herausgestellt-, daß es besonders zweckmäßig ist, absolutierte Lösungsmittel einzusetzen und die geringen Restmengen von Wasser zusammen mit dem immobilisierten Enzym in die Umsetzung einzubringen.

In besonders günstiger Verfahrensmodifikation wird die erfindungsgemäße Umsetzung so geführt, daß ein immobilisiertes Enzym eingesetzt wird, welches durch Beladung von in einem sauren Puffer gequollener Nitrocellulose mit der (S)-Oxynitrilase erhältlich ist.

In Weitergestaltung dieser Verfahrensvariante zeichnet sich das Verfahren der Erfindung dadurch aus, daß an Nitrocellulose immobilisierte (S)-Oxynitrilase eingesetzt wird, die durch Zugabe von (S)-Oxynitrilase zu in wäßriger, saurer Lösung vorgequollener Nitrocellulose, anschließendes Abfiltrieren der mit (S)-Oxynitrilase beladenen Nitrocellulose und Abzentrifugieren überschüssigen Wassers aus der beladenen Nitrocellulose erhältlich ist. Dabei wird unter "vorgequollener Nitrocellulose" ein Trägermaterial verstanden, welches beispielsweise in einem Citratpuffer bei pH 3,3 eine vorbestimmte Zeit quillt, dann vom Puffer abdekantiert wird und aus dem anschließend überschüssige flüssige Bestandteile abzentrifugiert werden. Das vorgequollene Material wird weiters im Vakuum getrocknet.

Für die Beladung des Trägermaterials mit dem Enzym hat es sich als besonders vorteilhaft herausgestellt, wenn diese bei einem pH-Wert im Bereich von ca. 3 bis 6 vorgenommen wird. Besonders bevorzugt ist der Bereich zwischen 3,3 und 5,5.

Erfindungsgemäß sind (S)-Oxynitrilasen verschiedener Herkunft mit Erfolg einsetzbar.

In einer besonders bevorzugten Variante handelt es sich bei der (S)-Oxynitrilase um ein Homomultimer mit einem nativen Molekulargewicht von 105 - 120 kDa. Weiters ist eine bevorzugte (S)-Oxynitrilase dadurch gekennzeichnet, daß sie als Homomultimer aus 30 kDa großen Untereinheiten zusammengesetzt ist.

Eine bevorzugte Quelle für ein solches Enzym ist Maniok. Das pH-Optimum des Enzyms aus Maniok [E.C. 4.1.2.37] liegt etwa im Bereich von 5,5. Dies entspricht fast exakt dem pH-Wert von 5,3, der in der Zelle vorliegt. Bei Untersuchungen zum Temperaturoptimum des Enzyms aus Maniok zeigt sich, daß die Aktivität bis 40 °C ständig ansteigt und zwischen 40 und 50 °C nahezu konstant bleibt.

Eine weitere Quelle für eine erfindungsgemäß einsetzbare (S)-Oxynitrilase stellt Hevea brasiliensis (Gummibaum) dar. Auch diese (S)-Oxynitrilase hat ein sehr breites Substratspektrum und setzt eine Vielzahl aliphatischer und aromatischer Carbonylverbindungen um.

Schließlich ist neben den Enzymen aus Manihot esculenta und Hevea brasiliensis auch jede zu diesen serologisch verwandte (S)-Oxynitrilase einsetzbar. Eine Beschreibung der (S)-Oxynitrilase isoliert aus Manihot esculenta findet sich beispielsweise in Plant Science 108 (1995) 1 - 11. Eine Beschreibung der (S)-Oxynitrilase isoliert aus Hevea brasiliensis ist beispielsweise in Plant Science 115 (1996) 25 - 31 angegeben.

Neben den bereits genannten Quellen ist eine im Rahmen der Erfindung einsetzbare (S)-Oxynitrilase in technischen Mengen leicht dadurch zugänglich, daß rekombinante (S)-Oxynitrilase beispielsweise aus Manihot esculenta eingesetzt wird. Durch die gentechnische Gewinnung der (S)-Oxynitrilase aus Manihot esculenta gelingt es, ein Enzym zu erschließen, das einerseits ein breites Substratspektrum zur Gewinnung von (S)-Cyanhydrinen aufweist und das andererseits aufgrund der Expressionsklonierung einfach und in ausreichenden Mengen zugänglich gemacht werden kann.

Insbesondere auch das rekombinante Enzym katalysiert die enantioselektive Addition von Blausäure an eine Vielzahl von Aldehyden und Ketonen. Blausäure kann dabei in direkter Form oder in Form einer Vorstufe, die unter den Bedingungen der Reaktion Blausäure freisetzt, eingesetzt werden. Eine durch Enzyme katalysierte enantioselektive Addition von HCN an Ketone konnte bisher für die Hydroxynitril-Lyase aus Bittermandeln (PaHNL) und für die Hydroxynitril-Lyase aus Linum usitatissimum, die beide die Bildung von (R)-Ketocyanhydrinen katalysieren, nachgewiesen werden. Eine Synthese von (S)-Ketocyanhydrinen mit einer (S)-Hydroxynitril-Lyase ist noch nicht beschrieben worden. (S)-Ketocyanhydrine konnten in jüngster Zeit durch Transcyanierung aus racemischen Ketocyanhydrinen mit der (R)-Hydroxynitril-Lyase PaHNL hergestellt werden. Es ist auffällig, daß die optischen Ausbeuten bei den Umsetzungen von Alkylmethylketon mit zunehmender Größe des Alkylrestes zunehmen. Während Verzweigungen in β-Position des Alkylsubstituenten keinen nachteiligen Einfluß auf die Enantioselektivität haben, nehmen bei starker sterischer Hinderung in Nachbarstellung zur Carbonylgruppe die optischen Ausbeuten stark ab. Die Hydroxynitril-Lyase aus Manihot esculenta weist damit ein überaus breites Substratspektrum auf, wobei ein aromatisches Keton wie Acetophenon problemlos als Substrat akzeptiert wird.

Nachfolgend wird die Erfindung anhand von Ausführungsbeispielen eingehender erläutert.

### Überexpression der MeHNL in E. coli

Die codierende Region des MeHNL-Gens wurde aus oligo(dT) geprimter cDNA mit'PCR amplifiziert ("Sense"-Primer: GCA GGG CCG GAT CCC ATT TCC AAA ATG GTA ACT GCA CA; "Antisense"-Primer: GCA GGG CCG GAT CCA CAC AAC GTG GAA CTC TCC CAT ATT; unterstrichene Bereiche entsprechen Position 16-39 bzw. 933-910 der cDNA-Sequenz der MeHNL) und im korrekten Leseraster in den pQE4-Expressionsvektor (Quiagen) kloniert. Das so erhaltene Expressionsplasmid pQE4-MeHNLwt wurde zur MeHNL-Expression in E. coli-M15[pREP4]-Zellen transfiziert (M15-MeHNL).

Eine 8 L-Kultur wurde mit 2 mL einer ca. 12 h alten Kultur von M15-MeHNL in LB-Medium mit Ampicillin (100 µg mL⁻¹) und Kanamycin (25 µg mL⁻¹) angeimpft. Diese Kultur wurde nach ca. 12 h Kultivierung bei 37 °C zum Animpfen eines 100 L-Fermenters (Bioengineering) verwendet. Die M15-MeHNL-Zellen wurden 1 h bei 30 °C und nach Induktion der Expression mit Isopropyl-β-D-thiogalactosid (IPTG) (Endkonzentration 1 mM) weitere 3,5 h kultiviert. Dann wurden die Zellen durch "Cross-flow"-Konzentrierung (Maxisette System) an einer 0,3 µm-Membran (0,46 m²) (Filtron) auf ca. 7,5 L eingeengt. Zur Abtrennung von restlichem LB Medium wurden die Zellen 10 min bei 10 000 x g zentrifugiert. Das erhaltene Zellpellet wurde in 2 L Natriumacetat-Puffer (50 mM, pH 5,4) resuspendiert und die Zellen durch Hochdruck-Homogenisation (500 bar, 3 Cyclen, Rannie-APV MiniLab) aufgeschlossen. Zur Entfernung chromosomaler DNA wurde das Rohlysat (Gesamtmenge an HNL 40 000 U, spez. Aktivität 2,8 U mg⁻¹) mit Benzonase (Merck) (Endkonzentration 5000 U L⁻¹) 1 h bei Raumtemperatur verdaut. Nach 1 h Zentrifugieren bei 130 000 x g wurde die MeHNL durch Anionenaustauschchromatographie auf eine spezifische Aktivität von 12 U mg⁻¹ angereichert. Hierzu wurde eine Q-Sepharose FF 100/1200 Säule mit 20 mM Natriumacetat-Puffer, pH 5,7, (Puffer A) equilibriert, und die gebundenen Proteine wurden mit 7200 mL eines linearen Gradienten von 0 - 1 M NaCl in Puffer A mit einer Flußrate von 50 mL min⁻¹ eluiert. MeHNL eluiert nach 450 mL Salzgradient.

### Reagenzien

a) 20 mM Natriumacetat-Puffer:
   0,57 ml Essigsäure (10 mmol) in 500 ml keimfreiem Wasser mit konzentrierter Natronlauge auf den gewünschten pH eingestellt.
   0,1 M Natriumacetat-Puffer pH 5,4:
      2,86 ml Essigsäure (50 mmol) in 497 ml keimfreiem Wasser mit konzentrierter Natronlauge auf pH 5,4 eingestellt.
   20 mM Natriumcitrat-Puffer mit pH 3,3:
      2,1 g Citronensäure-Monohydrat (10 mmol) in 500 ml keimfreiem Wasser gelöst und mit konzentrierter Natronlauge auf pH 3,3 eingestellt.
   10 %ige Acetoncyanhydrin-Lösung in 0,1 M Citronensäure:
      1 ml frisch destilliertes Acetoncyanhydrin gibt man zu 9 ml einer Citronensäure-Lösung (0,21 g Citronensäure-Monohydrat in 10 ml keimfreiem Wasser)
b) Lösungsmittel:
   Diisopropylether, Diethylether: Destillation über Natriumdraht
   Methylenchlorid: Destillation über Calciumhydrid
   Pyridin: Destillation über Kaliumhydroxid
   Acetanhydrid: Destillation
c) Edukte:
   Die Ausgangsverbindungen wurden von den genannten Firmen bezogen bzw. nach Autorenkollektiv, Organikum, VEB Verlag der Wissenschaften, Berlin, 16. Auflage (1986) hergestellt:
   Aldrich Chemie, Steinheim
   Fluka Chemika, Buchs (CH)
   Janssen Chimica, Belgien
   Merck-Suchhardt, Hohenbrunn

   Die Aldehyde und Ketone wurden jeweils frisch destilliert eingesetzt.
   racemische Cyanhydrine 3, 5: nach Autorenkollektiv, Organikum, VEB Verlag der Wissenschaften, Berlin, 16. Auflage (1986)
   wasserfreie Blausäure (2):
      durch Eintropfen von konzentrierter Natriumcyanid-Lösung in Schwefelsäure; die entstehende Blausäure wird bei -12 °C kondensiert und in Trockeneis gelagert.
   (S)-/(R)-MTPA-Cl: nach J. A. Dale, D. L. Dull, H. S. Mosher, J. Org. Chem. 34 (1969), 2543
   Spectroquant® 14800 CN⁻: Merck, Darmstadt
d) Trägermaterialien
   Avicel-Cellulose: Merck, Darmstadt
   P100PSC-Cellulose: Degussa, Frankfurt
   Nitrocellulose: Blotting-Membrane; Schleicher & Schuell, Dassel
e) Isolierung der (S)-Oxynitrilase [E.C. 4.1.2.37]:
   Die Isolierung der (S)-Oxynitrilase erfolgt einerseits aus gefriergetrockneten Maniokblättern aus Kolumbien, andererseits aus rekombinantem Protein aus E.coli mittels Ionenaustauschchromatographie.

### Enzym-katalysierte Darstellung von (S)-Cyanhydrinen

Man läßt 50 mg Träger (Nitrocellulose) in 3 mL 0,02 M Natriumcitrat-Puffer (pH 3,3) 30 min quellen. Nach Dekantieren, Zentrifugieren (30 min, 5700 x g) und 5 h Trocknen im Hochvakuum tropft man die in den Tabellen angegebene Menge konz. MeHNL-Lösung (900 U mL⁻¹) zu und zentrifugiert nach 15 min (bei -5 °C, 30 min 3650 x g). Man überführt den enzymbeladenen Träger in einen Kolben, gibt 5 mL Diisopropylether, 0,3 - 0,4 mmol 1 oder 3 und 100 µl (2,6 mmol) HCN zu und läßt die in den Tabellen angegebene Zeit bei Raumtemperatur rühren. Man saugt den Träger ab, wäscht mit Diethylether, trocknet die vereinigten Filtrate und destilliert das Lösungsmittel und nicht umgesetztes Edukt (1a-g, 3a-f) ab. Die Aldehydcyanhydrine (S)-2a-j (Tabelle 1) und die Ketoncyanhydrine (S)-4a-f (Tabelle 2) fallen in reiner Form an. Im Falle der Verbindungen (S)-2k-o und (S)-4g erfolgt die Reindarstellung über eine Derivatisierung als Acetate bzw. Trimethylsilylether, wobei die NMR-spektroskopisch ermittelten Ausbeuten (Tabelle 1, 2) bestätigt werden.

### Tabelle 1 und Schema 1:

(S)-Cyanhydrine (S)-2 durch MeHNL-katalysierte Addition von HCN an Aldehyde 1 in Diisopropylether als Lösungsmittel

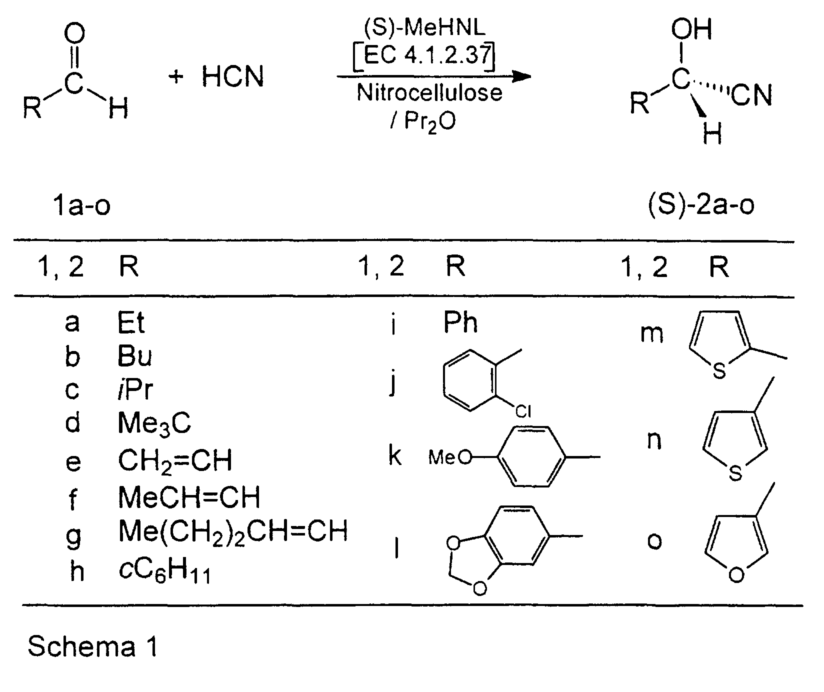

**Tabelle 1:**

| Aldehyde 1 | | Enzym (U/mmol 1) | (S)-Cyanhydrine 2 | | |
|---|---|---|---|---|---|
| | R | | *t*[h] | Ausb.[%][a] | *ee*[%][b] |
| a | C₂H₅ | 34 | 4.3 | 86 | 91 |
| b | *n*C₄H₉ | 61 | 4.0 | 100 | 91 |
| c | (H₃C)₂CH | 32 | 6.5 | 91 | 95 |
| d | (H₃C)₃C | 39 | 8.8 | 80 | 94 |
| e | H₂C=CH | 119 | 0.5 | 100 | 47 |
| f | H₃CCH=CH | 145 | 1.0 | 100 | 92 |
| g | *E*-H₃C(CH₂)₂CH=CH | 130 | 3.0 | 82 | 97 |
| h | *c*C₆H₁₁ | 70 | 5.3 | 100 | 92 |
| i | C₆H₅ | 58 | 7.0 | 100 | 98 |
| j | 2-Cl-C₆H₄ | 107 | 8.7 | 100 | 92 |
| k | 4-H₃CO-C₆H₄ | 130 | 9.5 | 82 | 98 |
| l | 3,4-CH₂O₂-C₆H₃ | 110 | 10.3 | 84 | 86 |
| m | 2-Thienyl | 134 | 6.0 | 85 | 96 |
| n | 3-Thienyl | 149 | 4.0 | 98 | 98 |
| o | 3-Furyl | 141 | 6.5 | 98 | 92 |

| | | | | | |
|---|---|---|---|---|---|
| [a] Ausbeute für 2h-o ¹H-NMR-spektroskopisch bestimmt. | | | | | |
| [b] Nach Acetylierung mit Acetanhydrid gaschromatographisch auf β-Cyclodextrinphasen bestimmt [1,9]. | | | | | |

### Tabelle 2 und Schema 2:

(S)-Ketoncyanhydrine (S)-4 durch MeHNL-katalysierte Addition von HCN an Methylketone 3 in Diisopropylether

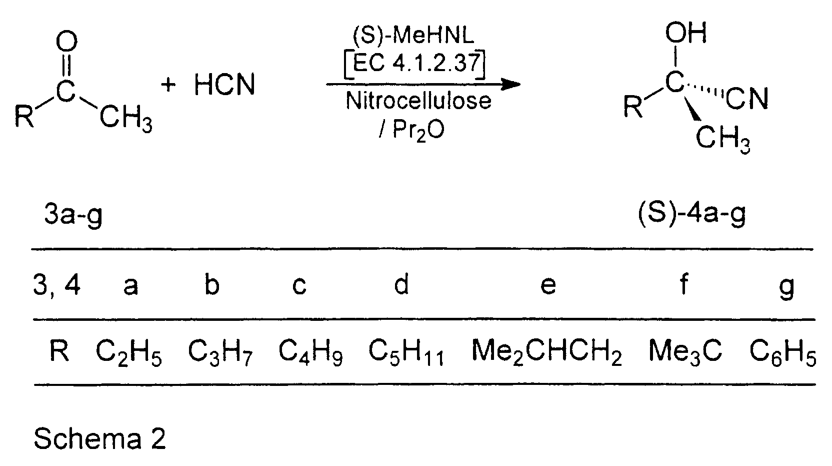

**Tabelle 2:**

| Ketone 3 | | Enzym (U/mmol 3) | (S)-Ketoncyanhydrine 4 | | |
|---|---|---|---|---|---|
| | R | | *t*[h] | Ausb.[%] | *ee*[%][a] |
| a | C₂H₅ | 53 | 4.0 | 91 | 18 |
| b | *n*C₃H₇ | 123 | 0.5 | 36 | 69 |
| c | *n*C₄H₉ | 123 | 0.5 | 58 | 80 |
| d | *n*C₅H₁₁ | 126 | 2.0 | 39 | 92 |
| e | (H₃C)₂CHCH₂ | 107 | 0.7 | 69 | 91 |
| f | (H₃C)₃C | 107 | 0.8 | 81 | 28 |
| g | C₆H₅ | 112 | 7.0 | 13[b] | 78 |

| | | | | | |
|---|---|---|---|---|---|
| [a] Nach Acetylierung mit Acetanhydrid, Verseifung zur Carbonsäure und anschließender Veresterung zum Methylester oder über diastereomere (S)-MTPA-Ester gaschromatographisch auf β-Cyclodextrinphasen bestimmt [1,9] | | | | | |
| [b] Ausbeute ¹H-NMR-spektroskopisch bestimmt. | | | | | |

### Trägervariation

Um die Vorteile gegenüber der seither verwandten Cellulose P100PSC herauszuarbeiten, werden die Träger am Beispiel der Umsetzung von Isobutyraldehyd (1e) miteinander verglichen.

**Tabelle 3:**

| Umsetzungen des Isobutyraldehyd (1c) mit (S)-Oxynitrilase und Blausäure (2) zum (S)-Cyanhydrin 2c unter Variation des Trägermaterials und zugehörige Blindproben | | | | | | |
|---|---|---|---|---|---|---|
| Träger | U/mmol 1e ¹⁾ | abzentrifug. | Rkt. - dauer | Blindprobe ²⁾ | (S)-Cyanhydrin 2c | |
| | | Enzymmenge [%] | [h] | Ausbeute [%] | Ausbeute [%] | ee(S)-2c [%] |
| 200 mg P100PSC in NaOAc pH 3,3 gequollen | 56 | -³⁾ | 7,5 | 71 | 54 | 49 |
| 150 mg P100PSC in NaOAc pH 3,3 gequollen, abzentrifugiert | 32 | 75 | 6,5 | 19 | 53 | 63 |
| 50 mg Nitrocellulose ungequollen, abzentrifugiert | 32 | 1 | 6,5 | 96 | 90 | 86 |
| 50 mg Nitrocellulose in Cit.-Puffer pH 3,3 gequollen, abzentrifugiert | 32 | 2 | 6,5 | 17 | 91 | 95 |

| | | | | | | |
|---|---|---|---|---|---|---|
| ¹⁾ eingesetzte Enzymmenge vor dem Abzentrifugieren | | | | | | |
| ²⁾ anstatt der Enzymlösung verwendet man eine entsprechende Menge eines 20 mM Natriumacetat-Puffers pH 5,4 | | | | | | |
| ³⁾ wurde nicht abzentrifugiert | | | | | | |

Betrachtet man die beiden Ansätze mit der Cellulose P100PSC, so sieht man, daß beim Abzentrifugieren 75 % des Enzyms ins Zentrifugat geht. Deshalb steigt der Enantiomerenübeschuß trotz sehr viel geringerem Wassergehalt nur geringfügig an. Im Gegensatz dazu bleibt die (S)-Oxynitrilase beim Zentrifugieren nahezu quantitativ auf der Nitrocellulose. Wegen der hohen Affinität zu Proteinen wird hier also lediglich Wasser abzentrifugiert. Der niedrige Wassergehalt ist ausschlaggebend für den deutlichen Anstieg der optischen Ausbeuten.

Die besten Ergebnisse bezüglich der chemischen und optischen Ausbeuten erzielt man mit in Natriumcitrat-Puffer pH 3,3 vorgequollener Nitrocellulose, die anschließend abzentrifugiert wird. Hier hat man die beiden Vorteile der geringen Wassermenge und des niedrigen pH-Wertes kombiniert. Dies zeigt sich sowohl im sehr hohen Enantiomerenüberschuß für das Cyanhydrin 2c, als auch in der relativ geringen Ausbeute der Blindprobe.

**Tabelle 4:**

| Vergleich der Träger bei Verwendung einer konzentrierten Enzymlösung (900 U/ml) | | | | | |
|---|---|---|---|---|---|
| Substrat | Rkt.-dauer | P100PSC | | Nitrocellulose | |
| | [h] | Ausbeute/Umsatz | ee[%] | Ausbeute/Umsatz | ee[%] |
| Heptanal | 9 | 69 | 79 | 61 | 79 |
| Acrolein | 1 | 29 | 41 | 70 | 56 |
| Furfural | 9 | 68 | 89 | 84 | 97 |
| 3-Methoxybenzaldehyd | 8,5 | 25 | 92 | 32 | 99 |

## Patentansprüche

1. Verfahren zur Herstellung von (S)-Cyanhydrinen der allgemeinen Formel I 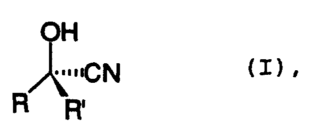 worin die Reste R und R' jeweils unabhängig voneinander bedeuten:
- Wasserstoff;
- einen substituierten oder unsubstituierten, linearen oder verzweigten, gesättigten Alkylrest mit 1 bis 18 C-Atomen, der als Substituenten einen oder mehrere Amin-, Imin-, Hydroxy-, C₁-C₈-Alkoxy, Halogen-, Carboxyl-, C₃-C₂₀-Cycloalkylrest und/oder einen ggf. N,O,S-heteroatomsubstituierten aromatischen Ring mit bis zu 22 C-Atomen aufweisen kann, wobei die zyklischen Substituenten selbst ein- oder mehrfach mit Halogen, Hydroxy und/oder linearen oder verzweigten C₁-C₈-Alkyl oder C₂-C₈-Alkenyl oder C₂-C₈-Alkinyl substituiert sein können;
- einen substituierten oder unsubstituierten, linearen oder verzweigten, ein- oder mehrfach ungesättigten Alkenyl- oder Alkinylrest mit 2 bis 18 C-Atomen, der als Substituenten einen oder mehrere Amin-, Imin-, Hydroxy-, C₁-C₈-Alkoxy, Halogen-, Carboxyl-, C₃-C₂₀-Cycloalkylrest und/oder einen ggf. N,O,S-heteroatomsubstituierten aromatischen Ring mit bis zu 22 C-Atomen aufweisen kann, wobei die zyklischen Substituenten selbst ein- oder mehrfach mit Halogen, Hydroxy und/oder linearen oder verzweigten C₁-C₈-Alkyl oder C₂-C₈-Alkenyl oder C₂-C₈-Alkinyl substituiert sein können;
- einen substituierten oder unsubstituierten aromatischen oder heteroaromatischen Rest mit 5 bis 22 Ringatomen, wobei bis zu 4 der Ringkohlenstöffatome durch N, O und/oder S ersetzt sein können, und wobei der Rest als Substituent einen oder mehrere Amin-, Imin-, Hydroxy-, C₁-C₈-Alkoxy-, Aryloxy-, Halogen-, Carboxy- und/oder linearen oder verzweigten, gesättigten oder einfach oder mehrfach ungesättigten Alkylrest mit ein bis 22 C-Atomen aufweisen kann, und wobei wenigstens zwei der Substituenten am Ring zu einem Zyklus zusammengeschlossen sein können;
mit der Maßgabe, daß R' und R nicht gleichzeitig Wasserstoff bedeuten;
durch enzymkatalysierte Umsetzung von Carbonylverbindungen der allgemeinen Formel II 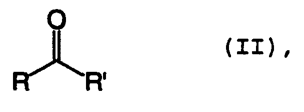 worin
R und R' die bei Formel (I) angegebene Bedeutung besitzen,
mit Blausäure oder einer Blausäure oder CN⁻ für die Umsetzung liefernden Substanz in Gegenwart einer die Umsetzung katalysierenden Menge eines immobilisierten Enzyms,
**dadurch gekennzeichnet,**
**daß** eine das, immobilisierte Enzym EC 4.1.2.37 aus Marihot esculenta tragende Nitrocellulose eingesetzt wird, die erhältlich ist durch Zugabe dieses Enzyms zu in wäßriger, saurer Lösung vorgequollener Nitrocellulose, Abfiltrieren der mit diesem Enzyms beladenen Nitrocellulose und Abzentrifugieren überschüssigen Wassers.

2. Verfahren nach Anspruch 1,
**dadurch gekennzeichnet,**
**daß** die enzymkatalysierte Umsetzung in einem organischen Lösungsmittel durchgeführt wird.

3. Verfahren nach Anspruch 2,
**dadurch gekennzeichnet,**
**daß** als organisches Lösungsmittel Diisopropylether eingesetzt wird.

4. Verfahren nach einem der Ansprüche 2 oder 3,
**dadurch gekennzeichnet,**
**daß** das organische Lösungsmittel Spuren von Wasser enthält.

5. Verfahren nach Anspruch 1,
**dadurch gekennzeichnet,**
**daß** die Beladung des Trägers mit dem Enzym bei einem pH-Wert im Bereich von ca. 3 - 6 vorgenommen wird.

6. Verfahren nach einem oder mehreren der vorhergehenden Ansprüche,
**dadurch gekennzeichnet,**
**daß** das eingesetzte Enzym sich als Homomultimer mit einem nativen Molekulargewicht von 105-102 kDa aus 30 KDa großen Untereinheiten zusammensetzt.

## Claims

1. Process for the preparation of (S)-cyanhydrins of the general formula I 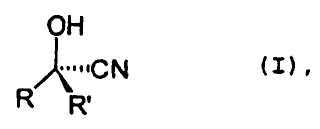 wherein the radicals R and R' in each case independently of one another denote:
- hydrogen;
- a substituted or unsubstituted, linear or branched, saturated alkyl radical with 1 to 18 C atoms that may carry as substituents one or more amine, imine, hydroxy, C₁-C₈-alkoxy, halogen, carboxyl, C₃-C₂₀-cycloalkyl radicals and/or an aromatic ring with up to 22 C atoms optionally substituted by the heteroatoms N, O, S, wherein the cyclic substituents may themselves be substituted singly or multiply by halogen, hydroxy and/or linear or branched C₁-C₈-alkyl or C₂-C₈-alkenyl or C₂-C₈-alkinyl;
- a substituted or unsubstituted, linear or branched, singly or multiply unsaturated alkenyl or alkinyl radical with 2 to 18 C atoms that may carry as substituents one or more amine, imine, hydroxy, C₁-C₈-alkoxy, halogen, carboxyl, C₃-C₂₀-cycloalkyl radicals and/or an aromatic ring with up to 22 C atoms that is optionally substituted by the heteroatoms N, O, S, wherein the cyclic substituents may themselves be substituted singly or multiply by halogen, hydroxy and/or linear or branched C₁-C₈-alkyl or C₂-C₈-alkenyl or C₂-C₈-alkinyl;
- a substituted or unsubstituted aromatic or heteroaromatic radical with 5 to 22 ring atoms, wherein up to 4 of the ring carbon atoms may be replaced by N, O and/or S, and wherein the radical may carry as substituent one or more amine, imine, hydroxy, C₁-C₈-alkoxy, aryloxy, halogen, carboxy and/or linear or branched, saturated or singly or multiply unsaturated alkyl-radicals with 1 to 22 C atoms, and wherein at least two of the substituents in the ring may be joined to form a cycle;
with the proviso that R' and R do not simultaneously denote hydrogen;
by enzyme-catalysed reaction of-carbonyl compounds of the general formula II 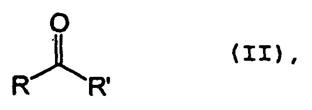 wherein
R and R' have the meanings specified in formula (I), with hydrocyanic acid or with a substance providing hydrocyanic acid or CN⁻ for the reaction, in the presence of an amount of an immobilised enzyme catalysing the reaction, **characterised in that** a nitrocellulose carrying the immobilised enzyme EC 4.1.2.37 from Manihot esculenta is used, which can be obtained by adding this enzyme to nitrocellulose preswollen in aqueous, acidic solution, filtering off the nitrocellulose charged with this enzyme, and centrifuging off excess water.

2. Process according to claim 1, **characterised in that** the enzyme-catalysed reaction is carried out in an organic solvent.

3. Process according to claim 2, **characterised in that** diisopropyl ether is used as organic solvent.

4. Process according to one of claims 2 or 3, **characterised in that** the organic solvent contains traces of water.

5. Process according to claim 1, **characterised in that** the charging of the carrier with the enzyme is carried out at a pH value in the range from ca. 3 to 6.

6. Process according to one or more of the preceding claims, **characterised in that** the enzyme that is used is an homomultimer with a natural molecular weight of 105-120 kDa, composed of 30 kDa large subunits.

## Revendications

1. Procédé pour la préparation de (S)-cyanhydrines de formule générale I 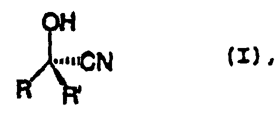 dans laquelle les radicaux R et R' signifient à chaque fois indépendamment l'un de l'autre :
- un atome d'hydrogène ;
- un radical alkyle substitué ou non substitué, linéaire ou ramifié, saturé, comprenant 1 à 18 atomes de carbone, qui peut présenter comme substituants un ou plusieurs radicaux amine, imine, hydroxy, alcoxy en C₁ à C₈, halogène, carboxyle, cycloalkyle en C₃ à C₂₀ et/ou le cas échéant un cycle aromatique substitué par un hétéroatome N, O, S comprenant jusqu'à 22 atomes de carbone, les substituants cycliques pouvant eux-mêmes être monosubstitués ou polysubstitués par halogène, hydroxy et/ou un alkyle en C₁ à C₈, alcényle en C₂ à C₈ ou alcynyle en C₂ à C₈, linéaires ou ramifiés;
- un radical alcényle ou alcynyle substitué ou non substitué, linéaire ou ramifié, monoinsaturé ou polyinsaturé, comprenant 2 à 18 atomes de carbone, qui peut présenter comme substituants un ou plusieurs radicaux amine, imine, hydroxy, alcoxy en C₁ à C₈, halogène, carboxyle, cycloalkyle en C₃ à C₂₀ et/ou un cycle aromatique le cas échéant substitué par un hétéroatome N, O, S, comprenant jusqu'à 22 atomes de carbone, les substituants cycliques pouvant eux-mêmes être monosubstitués ou polysubstitués par halogène, hydroxy et/ou un alkyle en C₁ à C₈, alcényle en C₂ à C₈ ou alcynyle en C₂ à C₈, linéaires ou ramifiés ;
- un radical aromatique ou hétéroaromatique substitué ou non substitué comprenant 5 à 22 atomes dans le cycle, jusqu'à 4 des atomes de carbone du cycle pouvant être remplacés par N, O et/ou S et le radical pouvant présenter comme substituant un ou plusieurs radicaux amine, imine, hydroxy, alcoxy en C₁ à C₈, aryloxy, halogène, carboxy et/ou alkyle linéaire ou ramifié, saturé ou monoinsaturé ou polyinsaturé comprenant jusqu'à 22 atomes de carbone et au moins deux des substituants sur le cycle peuvent être fermés en formant un cycle,
à condition que R et R' ne puissent pas signifier simultanément un atome d'hydrogène ;
par transformation catalysée par une enzyme de composés carbonyle de formule générale II 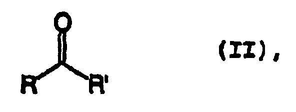 dans laquelle
R et R' présentent la signification indiquée pour la Formule (1),
avec de l'acide cyanhydrique ou un substituant libérant de l'acide cyanhydrique ou CN⁻ pour la transformation, en présence d'une quantité catalysant la transformation d'une enzyme immobilisée, **caractérisé en ce qu'**on utilise une nitrocellulose portant l'enzyme immobilisée EC 4.1.2.37 provenant de Marihot esculenta, qui peut être obtenue par addition de cette enzyme dans une solution aqueuse, acide de nitrocellulose gonflée au préalable, filtration de la nitrocellulose chargée de cette enzyme et élimination par centrifugation de l'eau en excès.

2. Procédé selon la revendication 1, **caractérisé en ce que** la transformation catalysée par une enzyme est réalisée dans un solvant organique.

3. Procédé selon la revendication 2, **caractérisé en ce qu'**on utilise du diisopropyléther comme solvant organique.

4. Procédé selon l'une quelconque des revendications 2 ou 3, **caractérisé en ce que** le solvant organique contient des traces d'eau.

5. Procédé selon la revendication 1, **caractérisé en ce que** la charge du support avec l'enzyme est réalisée à un pH dans la plage d'environ 3 à 6.

6. Procédé selon l'une ou plusieurs des revendications précédentes, **caractérisé en ce que** l'enzyme utilisée est constituée, en tant que homomultimère présentant un poids moléculaire de départ de 105 à 120 kDa, de sous-unités d'une taille de 30 kDa.
